(19)

Europäisches
Patentamt
European
Patent Office
Office européen
des brevets

(11) EP 2 143 384 A1

(12) **EUROPEAN PATENT APPLICATION**

(43) Date of publication:
13.01.2010 Bulletin 2010/02

(51) Int Cl.:
A61B 8/08 (2006.01)          G01S 15/89 (2006.01)
G06T 11/00 (2006.01)          G06T 15/00 (2006.01)
G06F 9/302 (2006.01)

(21) Application number: 09164024.3

(22) Date of filing: 29.06.2009

(84) Designated Contracting States:
AT BE BG CH CY CZ DE DK EE ES FI FR GB GR
HR HU IE IS IT LI LT LU LV MC MK MT NL NO PL
PT RO SE SI SK TR

(30) Priority: 09.07.2008 KR 20080066718
30.10.2008 KR 20080106811

(71) Applicant: MEDISON CO., LTD.
Gangwon-do 250-875 (KR)

(72) Inventors:
• Ahn, Chi Young
135-851, Seoul (KR)

• Lee, Suk Jin
135-851, Seoul (KR)
• Kim, Yung Gil
135-851, Seoul (KR)

(74) Representative: Schmid, Wolfgang
Lorenz & Kollegen
Patentanwälte Partnerschaftsgesellschaft
Alte Ulmer Straße 2
89522 Heidenheim (DE)

(54) **Enhanced ultrasound data processing in an ultrasound system**

(57) Embodiments for ultrasound data processing in an ultrasound system are disclosed. In one embodiment, a mapping table, in which linear operation information is associated with a plurality of diagnostic modes, is stored in a storage unit. If there is an instruction to select at least one of the diagnostic modes, then an ultrasound data acquisition unit transmits/receives ultrasound signals to/ from a target object to thereby acquire ultrasound data according to the selected diagnostic mode. A processing unit forms an ultrasound data matrix based on the ultrasound data and retrieves the linear operation information associated with the selected diagnostic mode from the mapping table to form linear operation matrices. Further, the processing unit performs a matrix operation of the ultrasound data matrix and the linear operation matrices.

FIG. 3

140

EP 2 143 384 A1

## Description

The present application claims priority from Korean Patent Application Nos. 10-2008-66718 and 10-2008-106811 filed on July 9, 2008 and October 30, 2008, respectively, the entire subject matters of which are incorporated herein by references.

## TECHNICAL FIELD

The present disclosure generally relates to ultrasound systems, and more particularly to enhanced ultrasound data processing in an ultrasound system.

## BACKGROUND

Recently, an ultrasound system has been extensively used in the medical field due to its non-invasive and non-destructive nature. Modem high-performance ultrasound imaging systems and techniques are commonly used to produce two dimensional ultrasound images and three-dimensional ultrasound images of internal features of patients.

Generally, to form the ultrasound images, time gain compensation, filtering (e.g., finite impulse response filtering), decimation, in-phase/quadrature-phase (I/O) data formation, compression, scan conversion and the like should be sequentially performed. Thus, problems arise since it takes a long time to form the ultrasound image data, which makes it difficult to increase a frame rate.

## SUMMARY

Embodiments for enhanced ultrasound data processing in an ultrasound system are disclosed herein. In one embodiment, by way of non-limiting example, an ultrasound system comprises: a storage unit for storing a mapping table having linear operation information associated with a plurality of diagnostic modes; an input unit for allowing a user to input an instruction to select at least one of the diagnostic modes; an ultrasound data acquisition unit operable to transmit/receive ultrasound signals to/from a target object according to the selected diagnostic mode to thereby acquire ultrasound data; and a processing unit operable to form an ultrasound data matrix based on the ultrasound data and retrieve the linear operation information associated with the selected diagnostic mode from the mapping table to form linear operation matrices, the processing unit being further operable to perform a linear operation upon the ultrasound data matrix and the linear operation matrices and perform at least one non-linear operation upon the ultrasound data.

In another embodiment, a method of processing ultrasound data in an ultrasound system, comprises: a) storing a mapping table associating linear operation information with a plurality of diagnostic modes; b) receiving an instruction to select at least one of the diagnostic mode; c) transmitting/receiving ultrasound signals to/from a target object to thereby acquire ultrasound data according to the selected diagnostic mode; and d) forming an ultrasound data matrix based on the ultrasound data, retrieving the linear operation information associated with the selected diagnostic mode from the mapping table to form linear operation matrices, and performing a linear operation upon the ultrasound data matrix and the linear operation matrices.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

## BRIEF DESCRIPTION OF THE DRAWINGS

FIG. 1 is a block diagram showing an ultrasound system in accordance with a first embodiment.

FIG. 2 is a block diagram showing an ultrasound data acquisition unit in accordance with a first embodiment.

FIG. 3 is a block diagram showing a processing unit in accordance with a first embodiment.

FIG. 4 is a block diagram showing an ultrasound system in accordance with a second embodiment.

FIG. 5 is a block diagram showing a processing unit in accordance with a second embodiment.

FIG. 6 is a block diagram showing an ultrasound system in accordance with a third embodiment.

FIG. 7 is a block diagram showing a first processing unit in accordance with a third embodiment.

FIG. 8 is a block diagram showing a second processing unit in accordance with a third embodiment.

FIG. 9 is a schematic diagram showing an example of vertex initialization in accordance with a third embodiment.

FIG. 10 is an exemplary diagram showing an example of forming texture data in accordance with a third embodiment.

FIG. 11 is a block diagram showing an ultrasound system in accordance with a fourth embodiment.

FIG. 12 is a block diagram showing a first processing unit in accordance with a fourth embodiment.

FIG. 13 is a block diagram showing an ultrasound system in accordance with a fifth embodiment.

FIG. 14 is a block diagram showing a processing unit in accordance with a fifth embodiment.

## DETAILED DESCRIPTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

First embodiment

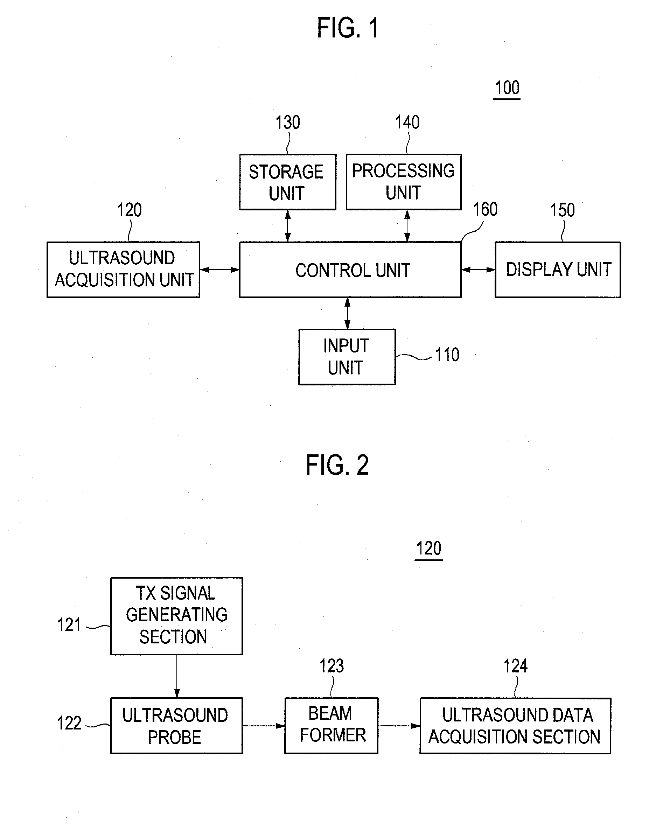

Referring to FIG. 1, an ultrasound system 100 may include an input unit 110 that may be operable to allow a user to input instructions. In one embodiment, the instructions may include an instruction for selecting one of diagnostic modes provided by the ultrasound system 100. The input unit 110 may include a control panel, a mouse, a keyboard, a track ball and the like. However, it is not limited thereto.

The ultrasound system 100 may further include an ultrasound data acquisition unit 120. The ultrasound data acquisition unit 120 may be operable to transmit/receive ultrasound signals to/from the target object, thereby outputting ultrasound data. The ultrasound data acquisition unit 120 may include a transmit (Tx) pulse signal generating section 121, as shown in FIG. 2. The Tx pulse signal generating section 121 may be operable to generate a plurality of Tx pulse signals. The ultrasound data acquisition unit 120 may further include an ultrasound probe 122 containing a plurality of elements for reciprocally converting ultrasound signals and electrical signals. The ultrasound probe 122 may be operable to transmit ultrasound signals into a target object in response to the Tx pulse signals. The ultrasound probe 122 may be further operable to receive echo signals reflected from the target object to thereby output electrical receive signals, which may be analog signals. In one embodiment, the ultrasound probe 122 may be a convex probe, a linear probe, a 3-dimensional probe, a trapezoidal probe, an IVUS probe or the like. However, it is not limited thereto.

The ultrasound data acquisition unit 120 may further include a beam former 123. The beam former may be operable to convert the electrical receive signals into digital signals. The beam former 123 may be further operable to apply delays to the digital signals in consideration of distances between the elements and focal points, thereby outputting digital receive-focused signals. In one embodiment, the beam former 123 may be configured with a field programmable gate array (FPGC) or an application specific integrated circuit (ASIC) to enhance a processing speed of the receive signals.

The ultrasound data acquisition unit 120 may further include an ultrasound data forming section 124 that may be operable to form the ultrasound data corresponding to at lease one frame of an ultrasound image based on the digital receive-focused signals. In one embodiment, the ultrasound image may include a brightness mode (B-mode), a Doppler mode (D-mode) image, a color (C) mode image, an elastic (E) mode image and a 3-dimensional (3D) mode image. The B-mode image may be represented with gray scales based on reflectivity of the ultrasound signals in the target object. The D-mode image may represent velocities of a moving object (e.g., blood flow) with spectral Doppler by using Doppler effect. The C-mode image may represent velocities of a moving object with colors and the E mode image may be obtained by using displacements of the target object before and after applying a pressure thereto. The 3D-mode may 3-dimensionally display an ultrasound image based on the reflectivity of ultrasound signals in the target object. Although it is described that the diagnostic mode includes the B-mode, D-mode, 3D-mode, C-mode and E-mode, the diagnostic mode is not limited thereto.

Referring again to FIG. 1, the ultrasound system 100 may further include a storage unit 130. The storage unit 130 may store the ultrasound data corresponding to at least one frame of an ultrasound image. The storage unit 130 may further store information upon linear operations (hereinafter, referred to as linear operation information) to be explained later. In one embodiment, the storage unit 130 may store a mapping table in which the linear operation information is associated with diagnostic modes as shown in Table 1.

[Table 1]

| Diagnostic mode | Linear operation information |
|---|---|
| B-mode | TGC parameters, decimation parameters, quadrature mixer parameters, FIR filter parameters, scan conversion parameters |
| D-mode | TGC parameters, decimation parameters, quadrature mixer parameters, FIR filter parameters, clutter filter parameters, FFT parameters, scan conversion parameters |
| C-mode | TGC parameters, decimation parameters, quadrature mixer parameters, FIR filter parameters, clutter filter parameters, scan conversion parameters |
| E-mode | TGC parameters, decimation parameters, quadrature mixer parameters, FIR filter parameters, scan conversion parameters |
| 3D mode | TGC parameters, decimation parameters, quadrature mixer parameters, FIR filter parameters, rendering parameters |

In one embodiment, the storage unit 130 may include a first storage section (not shown) for storing the ultrasound data corresponding to at least one frame of an ultrasound image and a second storage section (not shown) for storing the mapping table.

The ultrasound system 100 may further include a processing unit 140. The processing unit 140 may be operable to form a plurality of linear operation matrices to be used for performing linear operations (e.g., matrix multiplication) upon the ultrasound data. In one embodiment, the linear operations may include time gain compensation (TGC) for compensating for attenuation of the ultrasound signals in the target object, decimation for adjusting amount of the ultrasound data, quadrature mixing for forming in-phase/quadrature (I/Q) data, finite impulse response (FIR) filtering, clutter filtering, fast Fourier transform (FFT), scan conversion and interpolation, rendering and the like. However, they are not limited thereto. The linear operation information may be TGC parameters, decimation parameters, quadrature mixer parameters, FIR filter parameters, clutter filter parameters, FFT parameters, scan conversion parameters, rendering parameters and the like. The linear operation matrices may be formed by searching the mapping table in response to an instruction for selecting a desired diagnostic mode, which is inputted through the input unit 110. The processing unit 140 may be further operable to perform a plurality of linear operations corresponding to the selected diagnostic mode by using the linear operation matrices and perform non-linear operations upon the ultrasound data. In one embodiment, the non-linear operations may include envelope detection, compression, auto correlation, arctangent operation, a least mean square operation, a differential operation and the like, but are not limited thereto. The processing unit 140 may be configured with one of a central processing unit, FPAG, ASIC, a digital signal processing chip and the like.

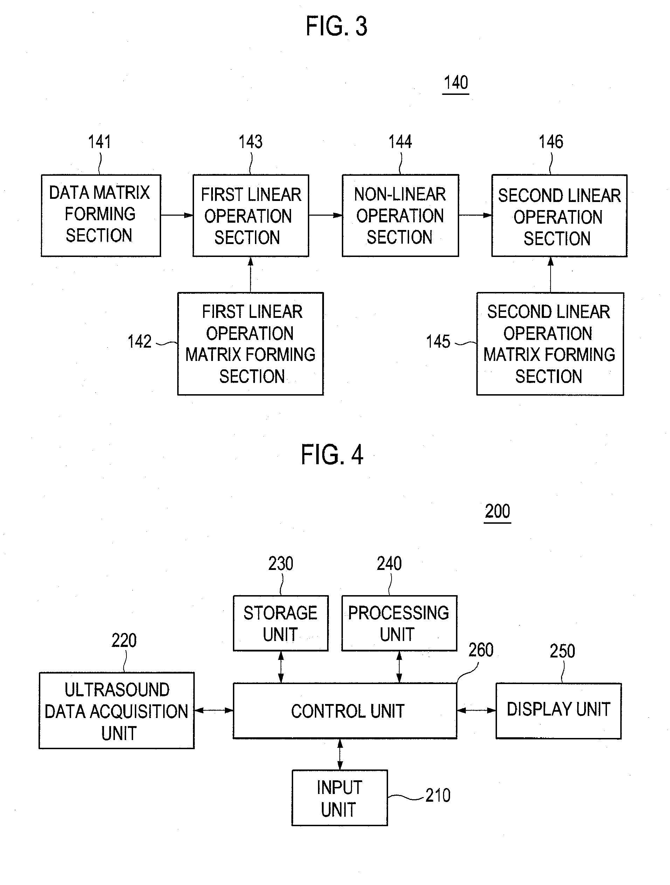

FIG. 3 is a block diagram showing an illustrative embodiment of the processing unit 140. As shown in FIG. 3, the processing unit 140 may include an ultrasound data matrix forming section 141. The ultrasound data matrix forming section 141 may be operable to form a first ultrasound data matrix based on the ultrasound data provided from the ultrasound data acquisition unit 120 or stored in the storage unit 130. In one embodiment, the ultrasound data may be obtained by scanning a plurality of scan lines and sampling electrical receive signals, which are outputted in response to echo signals reflected from the scan lines. That is, the ultrasound data may correspond to a plurality of sampling points on the scan lines. Thus, the first ultrasound data matrix may be formed to have a size of the number of sampling points on one scan line the number of the scan lines.

The processing unit 140 may further include a first linear operation matrix forming section 142. The first linear operation matrix forming section 142 may be operable to retrieve the linear operation information from the mapping table in response to an instruction for selecting a desired diagnostic mode, which is inputted through the input unit 110. The first linear operation matrix forming section 142 may be further operable to form a first linear operation matrix by using the retrieved linear operation information.

In one embodiment, if the instruction for selecting the B-mode is inputted through the input unit 110, the first linear operation matrix forming section 142 may be operable to retrieve the linear operation information associated with the B-mode from the mapping table, i.e., the TGC parameters, the decimation parameters, the quadrature mixer parameters and the FIR filter parameters. The first linear operation matrix forming section 142 may be operable to form a TGC matrix, a decimation matrix, a FIR filtering matrix and a quadrature mixing matrix by using the retrieved parameters. Each of the matrices may be multiply formed according to the necessity thereof. The size of each of the matrices may be changed depending on the resolution of the display unit 150. In one embodiment, the TGC matrix may be represented by a diagonal matrix, as follows:

$$\text{TGC matrix} = \begin{bmatrix} g_0 & 0 & 0 & 0 & \cdots & 0 \\ 0 & g_1 & 0 & 0 & \cdots & 0 \\ 0 & 0 & g_2 & 0 & \cdots & 0 \\ \vdots & \vdots & \vdots & \vdots & \ddots & \vdots \\ 0 & 0 & 0 & 0 & \cdots & g_{s-1} \end{bmatrix}$$

wherein $g_i$ is a TGC parameter and s is the number of the sample points.

The decimation matrix may be determined by decimating rows from a unit matrix according to a decimation rate. The decimation matrix may have a size of the number of sampling points after the decimation × the number of sampling points before the decimation. For example, when the decimation rate is 2, the decimation matrix may be determined as follows:

$$\text{Decimation matrix} = \begin{bmatrix} 1 & 0 & 0 & 0 & 0 & 0 & 0 & \cdots & 0 \\ 0 & 0 & 1 & 0 & 0 & 0 & 0 & \cdots & 0 \\ \vdots & & & & \vdots & & & & \vdots \\ \vdots & & & & \vdots & & & & \vdots \\ 0 & 0 & 0 & 0 & 1 & 0 & 0 & \cdots & 0 \\ 0 & 0 & 0 & 0 & 0 & 0 & 1 & \cdots & 0 \\ & & & & & & & \ddots & \end{bmatrix}$$

Also, when the decimation rate is 3, the decimation matrix may be determined as follows:

$$\text{Decimation matrix} = \begin{bmatrix} 1 & 0 & 0 & 0 & 0 & 0 & 0 & \cdots & 0 \\ 0 & 0 & 0 & 1 & 0 & 0 & 0 & \cdots & 0 \\ \vdots & & & \vdots & & & & & \vdots \\ \vdots & & & \vdots & & & & & \vdots \\ 0 & 0 & 0 & 1 & 0 & 0 & 0 & \cdots & 0 \\ 0 & 0 & 0 & 0 & 0 & 0 & 1 & \cdots & 0 \\ & & & & & & & \ddots & \end{bmatrix}$$

The FIR filtering matrix may be represented by a square band matrix, which has a bandwidth corresponding to the number of taps, as follows:

$$\text{FIR fitler matrix} = \begin{bmatrix} f_7 & \cdots & f_0 & 0 & \cdots & & \cdots & 0 \\ \vdots & \vdots & \vdots & f_0 & & & & \\ f_{14} & \vdots & \vdots & \vdots & \ddots & & & \vdots \\ 0 & f_{14} & \vdots & \vdots & & \ddots & & \\ \vdots & \vdots & \vdots & \vdots & \ddots & & \ddots & 0 \\ & & & & & \ddots & & f_0 \\ & & & & \ddots & & \ddots & \vdots \\ 0 & \cdots & & \cdots & 0 & f_{14} & \cdots & f_7 \end{bmatrix}$$

wherein $f_i$ is a FIR filter parameter.

The quadrature mixer matrix may be represented by a diagonal matrix, as follows:

$$\text{Quardrature mixer matrix} = \begin{bmatrix} q_0 & 0 & 0 & 0 & \cdots & 0 \\ 0 & q_1 & 0 & 0 & \cdots & 0 \\ 0 & 0 & q_2 & 0 & \cdots & 0 \\ \vdots & \vdots & \vdots & \vdots & \ddots & \vdots \\ 0 & 0 & 0 & 0 & \cdots & q_{s-1} \end{bmatrix}$$

wherein $q_i$ is a quardrature mixer parameter and s is the number of the sampling points before or after the decimation.

The first linear operation matrix forming section 142 may be operable to perform a linear operation (e.g., matrix multiplication) of the TGC matrix, the decimation matrix, the FIR filter matrix and the quadrature matrix to thereby form the first linear operation matrix.

If the instruction for selecting the D-mode is inputted through the input unit 110, then the first linear operation matrix forming section 142 may be operable to retrieve the linear operation information associated with the D-mode from the mapping table, i.e., the TGC parameters, the decimation parameters, the quadrature mixer parameters, the FIR filter parameters, the clutter filter parameters and the FFT parameters. The first linear operation matrix forming section 142 may be operable to form a TGC matrix, a decimation matrix, a FIR filter matrix, a quadrature matrix, a clutter filter matrix and a FFT matrix by using the retrieved parameters. The size of each of the matrices may be changed depending on the resolution of the display unit 150. In one embodiment, the clutter filter matrix may be represented by a Teoplitz matrix, as follows:

$$\text{Clutter filter matrix} = \begin{bmatrix} k_1 & 0 & 0 & 0 & \cdots & 0 \\ k_2 & k_1 & 0 & 0 & \cdots & 0 \\ \vdots & \vdots & \vdots & \vdots & \ddots & \vdots \\ k_N & & \cdots & & k_2 & k_1 \end{bmatrix}$$

wherein $k_i$ is a clutter filter parameter. In one embodiment, although the clutter filter is represented by the Teoplitz matrix, the clutter filter is not limited thereto.

Also, the FFT matrix may be represented as follows.

$$\text{FFT matrix} = \begin{bmatrix} 1 & 1 & \cdots & 1 \\ 1 & e^{-j2\pi 1/N} & \cdots & e^{-j2\pi(N-1)/N} \\ 1 & e^{-j2\pi 2/N} & \cdots & e^{-j2\pi 2(N-1)/N} \\ \vdots & \vdots & \vdots & \vdots \\ 1 & e^{-j2\pi(N-1)/N} & \cdots & e^{-j2\pi(N-1)^2/N} \end{bmatrix}$$

The first linear operation matrix forming section 142 may be operable to perform linear operations (e.g., matrix multiplication) of the TGC matrix, the decimation matrix, the FIR filter matrix, the quadrature mixer matrix, the clutter filter matrix and the FFT matrix to thereby form the first linear operation matrix.

If the instruction for selecting the C-mode is inputted through the input unit 110, then the first linear operation matrix forming section 142 may be operable to retrieve the linear operation information associated with the C-mode from the mapping table, i.e., the TGC parameters, the decimation parameters, the FIR filter parameter, the quadrature mixer parameters and the clutter filter parameters. The first linear operation matrix forming section 142 may be operable to form a TGC matrix, a decimation matrix, an FIR filter matrix, a quadrature matrix and a clutter filter matrix by using the retrieved parameters. In one embodiment, the clutter filter matrix may be determined to have a size of the ensemble number (or packet number), as follows:

$$\text{Clutter filter matrix} = \begin{bmatrix} c_{0,0} & c_{0,1} & \cdots & c_{0,n-1} \\ \vdots & \vdots & \ddots & \vdots \\ c_{n-1,0} & c_{n-1,1} & \cdots & c_{n-1,n-1} \end{bmatrix}$$

wherein $C_{ij}$ represents a clutter filter parameter and n is the ensemble number.

Although it is described above that the B-mode, the D-mode and the C-mode are performed, the E-mode or the 3D-mode may be also performed in the same manner as the B-mode, the D-mode and the C-mode.

The processing unit 140 may further include a first linear operation section 143. The first linear operation section 143 may be operable to perform a linear operation (e.g., matrix multiplication) of the first ultrasound data matrix and the first linear operation matrix, thereby outputting a second ultrasound data matrix. For example, assuming that the first data matrix is A and the first linear operation matrix is B, the first linear operation section 143 may be operable to perform the matrix operation of BxA.

The processing unit 140 may further include a non-linear operation section 144. The non-linear operation unit 144 may be operable to perform non-linear operations upon the ultrasound data contained in the second data matrix according to the selected diagnostic mode, thereby outputting a third ultrasound data matrix. In one embodiment, if the B-mode or the 3D-mode is selected, then the non-linear operation unit 144 may be operable to perform the non-linear operations (e.g., envelope detection and compression) upon the ultrasound data contained in the second data matrix, thereby outputting the third ultrasound data matrix. If the D-mode is selected, then the non-linear operation unit 144 may be operable to perform the non-linear operation (e.g., compression) upon the ultrasound data contained in the second data matrix, thereby outputting the third ultrasound data matrix. If the C-mode is selected, then the non-linear operation unit 144 may be operable to perform the non-linear operations (e.g., auto correlation and arctangent operation) upon the ultrasound data contained in the second data matrix, thereby outputting the third ultrasound data matrix. Further, if the E-mode is selected, then the non-linear operation unit 144 may be operable to perform the non-linear operations (e.g., auto correlation, least mean square operation and differential operation) upon the ultrasound data contained in the second data matrix, thereby outputting the third ultrasound data matrix.

The processing unit 140 may further include a second linear operation matrix forming section 145. The second linear operation matrix forming section 145 may be operable to perform linear operations upon the third ultrasound data matrix. The linear operations to be performed in the second linear operation matrix forming section 145 may include scan conversion and interpolation, rendering or the like. The second linear operation matrix forming section 145 may be operable to retrieve linear operation information associated with the selected diagnostic mode from the mapping table. The second linear operation matrix forming section 145 may be further operable to form a second linear operation matrix by using the retrieved linear operation information. In one embodiment, if one of the B-mode, D-mode, C-mode and E-mode is selected, then the second linear operation matrix forming section 145 may be operable to retrieve the scan conversion parameters from the mapping table.

The second linear operation matrix forming section 145 may be further operable to form a second linear operation matrix for scan conversion and interpolation of the third ultrasound data matrix by using the retrieved scan conversion parameters. The second linear operation matrix may be represented in consideration of a geometry of the ultrasound probe 122, as follows:

$$\text{Second linear operation matrix} = \begin{bmatrix} \alpha_{0,0} & \alpha_{0,1} & 0 & 0 & \cdots & 0 & 0 \\ 0 & \alpha_{1,1} & \alpha_{1,2} & 0 & \cdots & 0 & 0 \\ \vdots & \vdots & \vdots & \vdots & & \vdots & \vdots \\ 0 & 0 & 0 & 0 & \cdots & \alpha_{n-1,n-2} & \alpha_{n-1,n-1} \end{bmatrix}$$

Further, if the 3D-mode is selected in response to the instruction inputted through the input unit 110, then the second linear operation matrix forming section 145 may be operable to retrieve the rendering parameters from the mapping table. The second linear operation matrix forming section 145 may be further operable to form a second linear operation matrix for rendering upon the third data matrix by using the retrieved rendering parameters.

The processing unit 140 may further include a second linear operation section 146. The second linear operation section 146 may be operable to perform a linear operation (i.e., matrix multiplication) upon the third ultrasound data matrix and the second linear operation matrix. For example, assuming that the third ultrasound data matrix is C and the second linear operation matrix is D, the second linear operation section 146 may be operable to perform the matrix operation of DxC.

Referring again to FIG. 1, the ultrasound system 100 may further include a display unit 150. The display unit 150 may receive the ultrasound data from the processing unit 140 and display an ultrasound image based thereon.

The ultrasound system 100 may further include a control unit 160. The control unit 160 may be operable to control the transmission/reception of the ultrasound signals and the formation of the ultrasound data in the ultrasound data acquisition unit 120. The control unit 160 may be further operable to control the processing unit 140 to effect the linear operation and the non-linear operation of the ultrasound data. The control unit 160 may be also operable to control the display of the ultrasound image.

Although it is described above that the processing unit 140 sequentially performs the first linear operation, the non-linear operation and the second linear operation upon the ultrasound data, the order of the operations is not limited thereto. In another embodiment, the processing unit 140 may sequentially perform the first linear operation, the second linear operation and the non-linear operation upon the ultrasound data. Also, in another embodiment, the processing unit 140 may be further configured to sequentially perform the non-linear operation, the first linear operation and the second linear operation of the ultrasound data.

Further, in one embodiment, the second linear operation matrix forming section 145 may be configured to form the second linear operation matrix for performing the scan conversion in a row direction of the ultrasound data matrix. The second linear operation section 146 may be operable to perform the scan conversion of the ultrasound data matrix in a row direction thereof.

In another embodiment, the second linear operation matrix forming section 145 may be operable to form the second linear operation matrix for performing scan conversion of the ultrasound data matrix in a column direction thereof. The second linear operation section 146 may be further configured to perform the scan conversion of the ultrasound data matrix in a column direction thereof. In this case, the second linear operation matrix may be represented as the following matrix.

$$\text{Second linear operation matrix} = \begin{bmatrix} \beta_{1,0} & \beta_{1,1} & 0 & 0 & \cdots & 0 & 0 \\ \vdots & \vdots & \vdots & \vdots & & \vdots & \vdots \\ 0 & 0 & 0 & 0 & \cdots & \beta_{n-2,n-2} & \beta_{n-2,n-1} \\ 0 & 0 & 0 & 0 & \cdots & 0 & \beta_{n-1,n-1} \end{bmatrix}$$

Second embodiment

FIG. 4 is a block diagram showing an illustrative embodiment of the ultrasound system 200. As shown in FIG. 4, the ultrasound system 200 may include an input unit 210 that may be operable to allow a user to input instructions. The configuration and the operation of the input unit 210 may be identical to those of the input unit 110 of the first embodiment. Thus, detailed descriptions thereof will be omitted herein.

The ultrasound system 200 may include an ultrasound data acquisition unit 220 configured to transmit/receive ultrasound signals to/from a target object, thereby acquiring ultrasound data. The configuration and the operation of the ultrasound data acquisition unit 220 may be identical to those of the ultrasound data acquisition unit 120 of the first embodiment. Thus, detailed descriptions thereof will be omitted herein.

The ultrasound system 200 may further include a storage unit 230. The storage unit 230 may store the ultrasound data corresponding to at least one frame. The storage unit 230 may further store a mapping table in which information of linear operations is associated with diagnostic modes. The linear operations may be performed upon the ultrasound data to form an ultrasound image in each of the diagnostic modes. In one embodiment, the storage unit 230 may include a first storage section (not shown) for storing the ultrasound data and a second storage section (not shown) for storing the mapping table. The linear operation, the linear operation information and the mapping table may be identical to those described in the first embodiment. Thus, detailed descriptions thereof will be omitted herein.

The ultrasound system 200 may include a processing unit 240. The processing unit 240 may be operable to form a linear operation matrix to be used for performing a linear operation (e.g., matrix multiplication) upon the ultrasound data in response to the inputted instruction for selecting a desired diagnostic mode. The processing unit 240 may be further configured to perform the linear operation by using the linear operation matrix and the non-linear operation upon the ultrasound data. The non-linear operation may be identical to that of the first embodiment. Thus, detailed descriptions thereof will be omitted herein. The processing unit 140 may be configured with one of central processing unit, FPAG, ASIC, digital signal processing chip and the like to enhance the performance thereof.

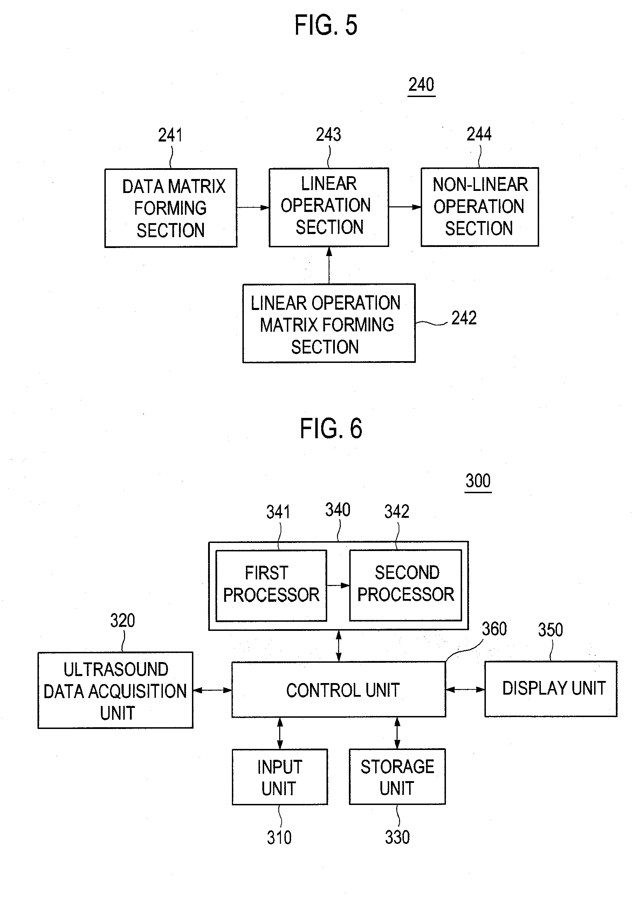

FIG. 5 is a block diagram showing an illustrative embodiment of the processing unit 240. Referring to FIG. 5, the processing unit 240 may include an ultrasound data matrix forming section 241, a linear operation matrix forming section 242, a linear operation section 243 and a non-linear operation section 244.

The ultrasound data matrix forming section 241 may be operable to form a first ultrasound data matrix by using the ultrasound data provided from the ultrasound data acquisition unit 220 or stored in the storage unit 230. Generally, the ultrasound data may be formed by scanning a plurality of scan lines and sampling electrical receive signals outputted in response to echo signals reflected from the scan lines. In one embodiment, since the ultrasound data may correspond to a plurality of sampling points on the scan lines, the first ultrasound data matrix may be formed to have a size of the number of the sampling points on one scan line the number of the scan lines..

The linear operation matrix forming section 242 may be operable to retrieve linear operation information corresponding to the diagnostic mode selected in response to the instruction inputted through the input unit 210 from the mapping table. The linear operation matrix forming section 242 may be further operable to form linear operation matrices by using the retrieved linear operation information.

In one embodiment, if the B-mode is selected, then the linear operation matrix forming section 242 may be operable to retrieve the linear operation information associated with the B-mode from the mapping table, i.e., the TGC parameters, the decimation parameters, the quadrature mixer parameters, the FIR filter parameters and the scan conversion parameters. The linear operation matrix forming section 242 may be operable to form a TGC matrix, a decimation matrix, an FIR filter matrix, a quadrature mixer matrix and a scan conversion matrix by using the retrieved parameters. The TGC matrix, the decimation matrices, the FIR filter matrices, the quadrature mixer matrix and the scan conversion matrix may be identically configured to those of the first embodiment. Thus, detailed descriptions thereof will be omitted herein.

If the D-mode is selected, then the linear operation matrix forming section 242 may be operable to retrieve the linear operation information associated with the D-mode from the mapping table, i.e., the TGC parameters, the decimation parameters, the quadrature mixer parameters, the FIR filter parameters, the clutter filter parameters, the FFT parameters and the scan conversion parameters. The linear operation matrix forming section 242 may be operable to form the TGC matrix, the decimation matrices, the FIR filter matrices, the quadrature matrix, a clutter filter matrix, a FFT matrix and the scan conversion matrix by using the retrieved parameters. The matrices formed in the linear operation matrix forming section 242 may be identically configured to those of the first embodiment. Thus, detailed descriptions thereof will be omitted herein.

Although it is described as examples that the B-mode and the D-mode are selected above, the selection of the diagnostic mode is not limited thereto. The C-mode, the E-mode or the 3D-mode may be also selected. In this case, the linear operation matrix forming section 242 may also retrieve the linear operation information corresponding to the selected diagnostic mode from the mapping table to thereby form the linear operation matrices.

The linear operation section 243 may be operable to perform a linear operation, e.g., matrix multiplication of the first ultrasound data matrix and the linear operation matrix, thereby outputting a second ultrasound data matrix. For example, assuming that the first ultrasound data matrix is E and the first linear operation matrix is F, the linear operation section 243 may be operable to perform the linear operation of FxE.

The non-linear operation unit 244 may be operable to perform the non-linear operation upon the ultrasound data contained in the second data matrix. In one embodiment, if the B-mode or the 3D-mode is selected, then the non-linear operation unit 244 may be operable to perform the non-linear operations (e.g., envelope detection and compression) upon the ultrasound data contained in the second data matrix. If the D-mode is selected, then the non-linear operation unit 244 may be operable to perform the non-linear operation (e.g., compression) upon the ultrasound data contained in the second data matrix. If the C-mode is selected, then the non-linear operation unit 244 may be operable to perform the non-linear operation (e.g., auto correlation and arctangent operation) upon the ultrasound data contained in the second data matrix. If the E-mode is selected, then the non-linear operation unit 244 may be operable to perform the non-linear operation (e.g., auto correlation, least mean square operation and differential operation) upon the ultrasound data contained in the second data matrix.

Referring again to FIG. 4, the ultrasound system 200 may further include a display unit 250. The display unit 250 may receive the ultrasound data with the linear and non-linear operations performed and display an ultrasound image based on the received ultrasound data.

The ultrasound system 200 may further include a control unit 260. The control unit 260 may be operable to control the transmission/reception of the ultrasound signals and the formation of the ultrasound data in the ultrasound data acquisition unit 220. The control unit 260 may be further operable to control the processing unit 240 to effect the linear operation and the non-linear operation upon the ultrasound data.
The control unit 260 may also control the display of the ultrasound image.

Although it is described that the processing unit 240 may be sequentially operable to perform the linear operation and the non-linear operation upon the ultrasound data in other embodiment, the processing unit 240 may be further operable to perform operations upon the ultrasound data in an order of the non-linear operation and the linear operation.

Third embodiment

FIG. 6 is a block diagram showing an illustrative embodiment of an ultrasound system 300. As depicted therein, the ultrasound system 300 may include an input unit 310 that may be operable to allow a user to input instructions. The configuration and the operation of the input unit 310 may be identical to those of the input unit 110 of the first embodiment. Thus, detailed descriptions thereof will be omitted herein.

The ultrasound system 300 may include an ultrasound data acquisition unit 320 that may be operable to transmit/receive ultrasound signals to/from a target object, thereby acquiring ultrasound data. The operation of the ultrasound data acquisition unit 320 may be identical to that of the ultrasound data acquisition unit 120 in the first embodiment. Thus, detailed descriptions thereof will be omitted herein.

The ultrasound system 300 may further include a storage unit 330. The storage unit 330 may store the ultrasound data corresponding to at least one frame. The storage unit 330 may further store a mapping table in which information of linear operations is associated with the diagnostic modes. The linear operation information may include TGC parameters for the TGC, decimation parameters for the decimation, quadrature mixer parameters for the quadrature mixing, FIR filter parameters for the FIR filtering, clutter filter parameters for the clutter filtering, FFT parameters for the FFT and the like. However, the linear operation information may not be limited thereto. In one embodiment, the storage unit 330 may store the mapping table as shown in Table 2.

[Table 2]

| Diagnostic mode | Linear operation information |
|---|---|
| B-mode | TGC parameters, decimation parameters, quadrature mixer parameters, FIR filter parameters |
| D-mode | TGC parameters, decimation parameters, quadrature mixer parameters, FIR filter parameters, clutter filter parameters, FFT parameters |
| C-mode | TGC parameters, decimation parameters, quadrature mixer parameters, FIR filter parameters, clutter filter parameters |
| E-mode | TGC parameters, decimation parameters, quadrature mixer parameters, FIR filter parameters |
| 3D mode | TGC parameters, decimation parameters, quadrature mixer parameters, FIR filter parameters |

In one embodiment, the storage unit 330 may include a first storage section (not shown) for storing the ultrasound data and a second storage section (not shown) for storing the mapping table.

The ultrasound system 300 may further include a processing unit 340. The processing unit 340 may be operable to form a linear operation matrix for performing linear operation upon the ultrasound data. In one embodiment, the linear operations may include time gain compensation (TGC), decimation for adjusting amount of the ultrasound data, quadrature mixing for forming in-phase/quadrature (I/Q) data, finite impulse response (FIR) filtering, clutter filtering, fast Fourier transform (FFT) and the like. However, the linear operations may not be limited thereto. The linear operation matrix may be formed by using the mapping table in response to an inputted instruction for selecting a desired diagnostic through the input unit 310. The processing unit 340 may be further configured to perform linear operations correspondding to the selected diagnostic mode by using the linear operation matrix and non-linear operation upon the ultrasound data. The non-linear operations may be identical to those described in the first embodiment. Thus, detailed descriptions thereof will be omitted herein. The processing unit 340 may be further operable to perform scan conversion or rendering upon the ultrasound data with the linear and non-linear operations performed. In one embodiment, the processing unit 340 may include a first processor 341 and a second processor 342.

The first processor 341 may be operable to form an ultrasound data matrix by using the ultrasound data. The first processor 341 may be operable to perform the linear and non-linear operations upon the ultrasound data matrix.

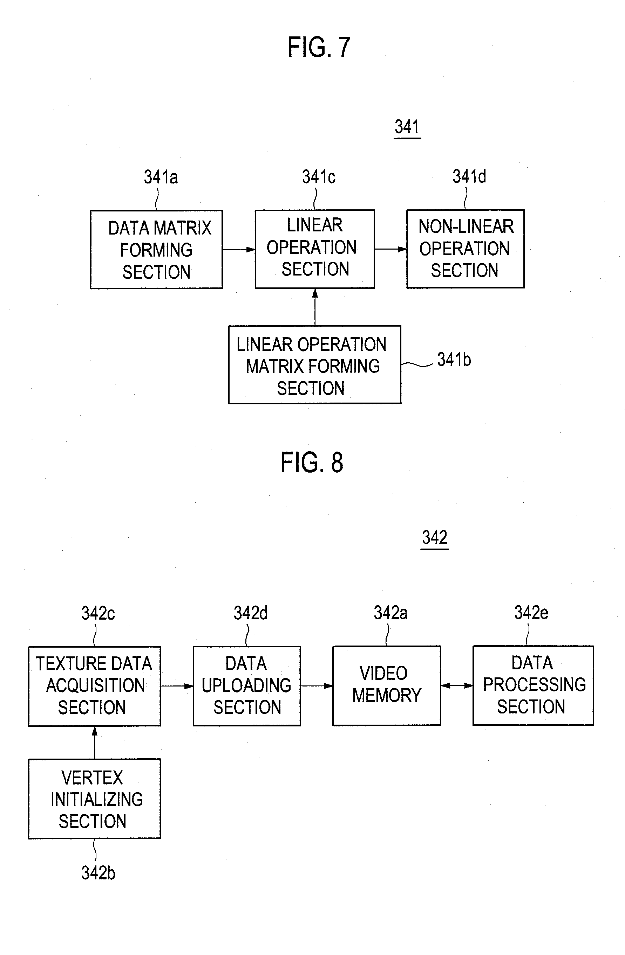

FIG. 7 is a block diagram showing an illustrative embodiment of the first processor 341. Referring to FIG. 7, the first processor 341 may include an ultrasound data matrix forming section 341a, a linear operation matrix forming section 341b, a linear operation section 341c and a non-linear operation section 341d.

The ultrasound data matrix forming section 341a may be operable to form a first ultrasound data matrix by using the ultrasound data provided from the ultrasound data acquisition unit 320 or stored in the storage unit 330. Generally, the ultrasound data may be formed by scanning a plurality of scan lines and sampling electrical receive signals outputted in response to echo signals reflected from the scan lines. In one embodiment, since the ultrasound data may correspond to a plurality of sampling points on the scan lines, the first ultrasound data matrix may be formed to have a size of the number of the sampling points on one scan line the number of the scan lines.

The linear operation matrix forming section 341b may be operable to retrieve linear operation information corresponding to the diagnostic mode selected in response to the instruction inputted through the input unit 210 from the mapping table stored in the storage unit 330. The linear operation matrix forming section 341b may be further operable to form a linear operation matrix by using the retrieved linear operation information.

In one embodiment, if the B-mode is selected, then the linear operation matrix forming section 341b may be operable to retrieve the linear operation information associated with the B-mode from the mapping table, i.e., the TGC parameters, the decimation parameters, the FIR filter parameters and the quadrature mixer parameters. The linear operation matrix forming section 341b may be operable to form a TGC matrix, a decimation matrix, an FIR filter matrix and a quadrature mixer matrix by using the retrieved parameters. The TGC matrix, the decimation matrices, the FIR filter matrices and the quadrature mixer matrix may be identical to those of the first embodiment. Thus, detailed descriptions thereof will be omitted herein.

If the D-mode is selected, then the linear operation matrix forming section 341b may be operable to retrieve the linear operation information associated with the D-mode from the mapping table, i.e., the TGC parameters, the decimation parameters, the FIR filter parameters, the quadrature mixer parameters, the clutter filter parameters and the FFT parameters. The linear operation matrix forming section 341b may be operable to form a TGC matrix, a decimation matrix, a FIR filter matrix, a quadrature matrix, a clutter filter matrix and a FFT matrix by using the retrieved parameters. The matrices formed in the linear operation matrix forming section 341b may be identical to those described in the first embodiment. Thus, detailed descriptions thereof will be omitted herein.

Although it is described as examples that the B-mode and the D-mode are selected above, the selection of the diagnostic mode is not limited thereto. The C-mode, the E-mode or the 3D-mode may be also selected. In this case, the linear operation matrix forming section 341b may also retrieve the linear operation information corresponding to the selected diagnostic mode from the mapping table to thereby form the linear operation matrix in the same manner as the B-mode or D-mode.

The linear operation section 341c may be operable to perform a linear operation (i.e., matrix operation) upon the first ultrasound data matrix and the linear operation matrix, thereby outputting a second ultrasound data matrix. For example, assuming that the first ultrasound data matrix is G and the first linear operation matrix is H, the linear operation section 341c may be operable to perform the matrix operation of $G \times H$.

The non-linear operation unit 341d may be operable to perform the non-linear operation upon the ultrasound data contained in the second data matrix. In one embodiment, if the B-mode or the 3D-mode is selected, then the non-linear operation unit 341d may be operable to perform the non-linear operations (e.g., envelope detection and compression) upon the ultrasound data contained in the second data matrix. If the D-mode is selected, then the non-linear operation unit 341d may be operable to perform the non-linear operation (e.g., compression) upon the ultrasound data contained in the second data matrix. If the C-mode is selected, then the non-linear operation unit 341d may be operable to perform the non-linear operations (e.g., auto correlation and arctangent operation) upon the ultrasound data contained in the second data matrix. If the E-mode is selected, then the non-linear operation unit 244 may be operable to perform the non-linear operations (e.g., auto correlation, least mean square operation and differential operation) upon the ultrasound data contained in the second data matrix.

Although it is described above that the non-linear operations are performed after performing the linear operations, the non-linear operations may be performed prior to the linear operations.

Referring to FIG. 6, the second processor 342 may be configured with a graphic processing unit (GPU) in one embodiment. The second processor 342 may be operable to perform a non-linear operation upon the ultrasound data based on the selected diagnostic mode.

FIG. 8 is a block diagram showing an illustrative embodiment of the second processor 342. Referring to FIG. 8, the second processor 342 may include a video memory 342a, a vertex initializing section 342b, a texture data forming section 342c, a data upload section 342d and a data processing section 342e.

The video memory 342a may include a plurality of storage areas including a storage area for the pixel data corresponding to pixels consisting of a screen of the display unit 350. That is, the pixel data formed in the second processor 342 may be stored at the corresponding storage area of the video memory 342a and displayed on a screen of the display unit 350 at the same time. Thus, the necessary capacity of the video memory 342a may depend on the number of pixels and data format for each of the diagnostic modes. For example, in case of an ultrasound image of 640 480 pixels, a minimum capacity of the video memory may be 640 480 8 bits. The data format may be different according to the diagnostic modes. For example, the B-mode image may have an 8-bit data format and the C-mode image may have a 16-bit data format.

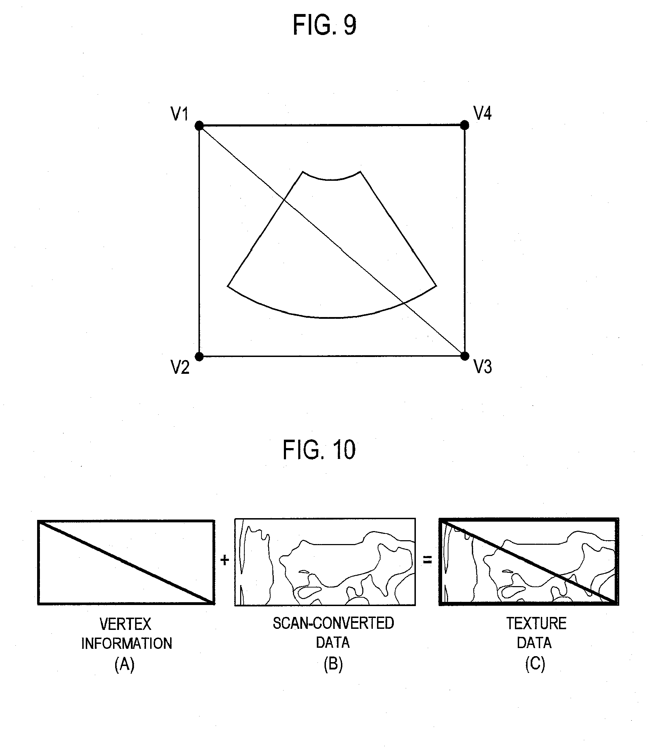

If the ultrasound data are inputted from the first processor 341, then the vertex initializing section 342b may be operable to form vertex information for initializing a position for displaying an ultrasound image on the screen of the display unit 350 by using a plurality of vertexes according to the selected diagnostic mode. The vertex initializing section 342b may be embodied with a 3-dimensiaonl application programming interface (3D API). Referring to FIG. 9, when the B-mode image is formed, the vertex initializing section 342b may be operable to form vertex information for initialize positioning of the vertexes V1, V2, V3 and V4. Although it is described that the vertexes V1, V2, V3 and V4 are set, the vertexes are not limited thereto. Other vertexes may be additionally set according to the necessity.

Textures of a 3D graphic format should be used to upload an ultrasound image to the video memory 342a. The texture data forming section 342c may be operable to combine vertex information "A" formed at the vertex initializing unit 33 with the ultrasound data "B" with the linear and non-linear operations processed according to the selected diagnostic mode to thereby form texture data C, as shown in FIG. 10. For example, when the B-mode image having an 8-bit data format is formed by using DirectX, texture data having a "D3DFMT_L8" format, which is the format only for an 8-bits, are formed. Also, when the C-mode image of a 16-bit data format is formed, texture data of a "D3DFMT_R5G6B5" format, which is a 16-bit only format, are formed.

The data upload section 342d may be operable to allocate the storage areas of the video memory 342a according to the data format corresponding to the selected diagnostic mode. In this case, the storage areas may include a storage area for storing the texture data and a storage area for storing code data (e.g., shader code, field shader code and color palette data in case of the C-mode) necessary for forming functions of the GPU. Also, the data upload section 342d may be further operable to upload the texture data and the code data to the corresponding storage areas in the video memory 342a.

The data processing section 342e may be operable to perform data processing (e.g., scan conversion or rendering) upon the texture data by using the shader codes uploaded to the video memory 342a according to the selected diagnostic mode. The data processing section 342e may be further operable to perform filtering, which applies the filter shader code to the scan-converted or rendered texture data, to thereby form the pixel data. The pixel data may be stored in a designated area in the video memory 342a.

Referring again to FIG. 6, the display unit 350 may display an ultrasound image based on the pixel data stored in the video memory 342a. The control unit 360 may be operable to control the transmission/reception of the ultrasound signals and the formation of the ultrasound data in the ultrasound data acquisition unit 320. The control unit 360 may be further operable to control the linear operation and the non-linear operation upon the ultrasound data in the processing unit 340. The control unit 360 may also control the display of the ultrasound image.

Fourth embodiment

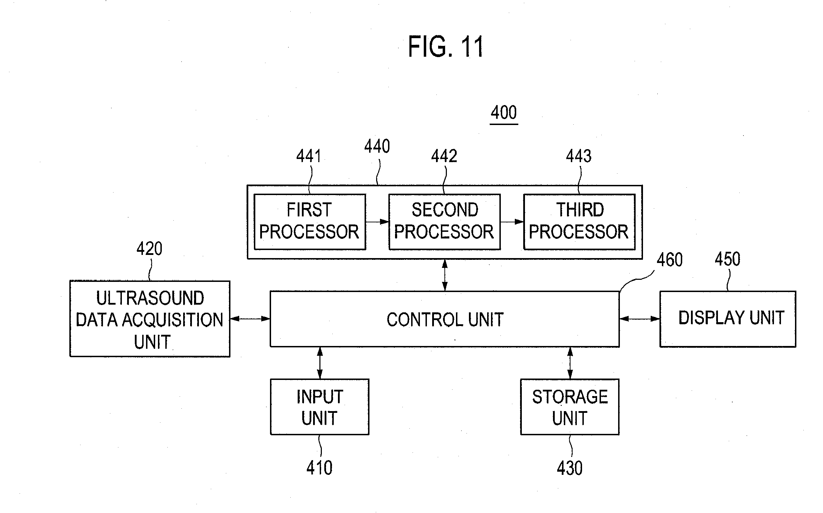

FIG. 11 is a block diagram showing an illustrative embodiment of an ultrasound system 400. The ultrasound system 400 may include an input unit 410, an ultrasound data acquisition unit 420, a storage unit 430, a processing unit 440, a display unit 450 and a control unit 460. The configurations and operations of the input unit 410 and the ultrasound data acquisition unit 420 may be identical to those of the input unit 110 and the ultrasound acquisition unit 120 of the first embodiment. As such, detailed descriptions thereof will be omitted herein. Also, the configuration of the storage unit 430 may be identical to that of the storage unit 330 of the third embodiment. Thus, the detailed description thereof will be omitted.

The processing unit 440 may be operable to form a linear operation matrix for performing linear operation upon the ultrasound data. The linear operation matrix may be formed by using the mapping table in response to an inputted instruction for selecting a desired diagnostic through the input unit 410. The processing unit 440 may be further configured to perform linear operations corresponding to the selected diagnostic mode by using the linear operation matrix and non-linear operation upon the ultrasound data. The non-linear operations may be identical to those described in the first embodiment. Thus, detailed descriptions thereof will be omitted herein. The processing unit 440 may be further operable to perform scan conversion or rendering upon the ultrasound data with the linear and non-linear operations performed. In one embodiment, the processing unit 440 may include a first processor 441, a second processor 442 and a third processor 443.

The first processor 441 may be embodied with a graphic processing unit (GPU) and be operable to form an ultrasound data matrix by using the ultrasound data. The first processor 441 may be operable to retrieve the linear operation information corresponding to the selected diagnostic mode in response to the instruction inputted through the input unit 410. The first processor 441 may be operable to form linear operation matrices perform linear operations upon the ultrasound data by using the retrieved linear operation information.

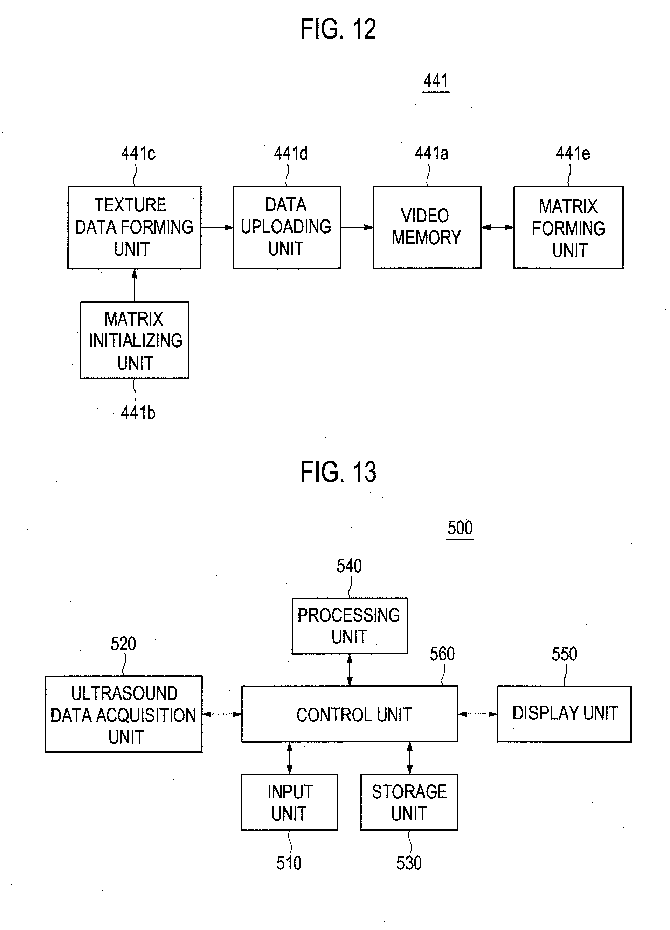

FIG. 12 is a block showing an illustrative embodiment of the first processor 441. Referring to FIG. 12, the first processor 441 may include a video memory 441a, a matrix initializing section 441b, a texture data forming section 411c, a data upload section 441d and a matrix forming section 441e. In one embodiment, the configuretion of the video memory 441a may be identical to that of the video memory 342a of the third embodiment. Thus, detailed description thereof will be omitted herein.

The matrix initializing section 441b may be operable to compute a size and elements of an ultrasound data matrix to be formed by using the ultrasound data, thereby forming first matrix information including the computed size and elements. Generally, the ultrasound data may be formed by scanning a plurality of scan lines and sampling electrical receive signals outputted in response to echo signals reflected from the scan lines. In one embodiment, since the ultrasound data may correspond to a plurality of sampling points on the scan lines, the ultrasound data matrix may be formed to have a size of the number of the sampling points the number of the scan lines.

The matrix initializing section 441b may be operable to retrieve linear operation information corresponding to the diagnostic mode selected in response to the instruction inputted through the input unit 410 from the mapping table stored in the storage unit 430. The matrix initializing section 441b may be further operable to compute a size and elements of a linear operation matrix to be formed by using the retrieved linear operation information, thereby forming second matrix information containing the computed size and elements.

In one embodiment, if the B-mode is selected, then the matrix initializing section 441b may be operable to retrieve the linear operation information associated with the B-mode from the mapping table, i.e., the TGC parameters, the decimation parameters, the FIR filter parameters and the quadrature mixer parameters. The matrix initializing section 41b may be operable to calculate sizes and elements of a TGC matrix, a decimation matrix, an FIR filter matrix and a quadrature mixer matrix to be formed by using the retrieved parameters, thereby forming the second matrix information including the computed sizes and elements. The TGC matrix, the decimaltion matrices, the FIR filter matrices and the quadrature mixer matrix may be identically established with those of the first embodiment. Thus, detailed descriptions thereof will be omitted herein.

If the D-mode is selected, then the matrix initializing section 441b may be operable to retrieve the linear operation information associated with the D-mode from the mapping table, i.e., the TGC parameters, the decimation parameters, the FIR filter parameters, the quadrature mixer parameters, the clutter filter parameters and the FFT parameters. The matrix initializing section 441b may be operable to compute sizes and elements of a TGC matrix, a decimation matrix, a FIR filter matrix, a quadrature matrix, a clutter filter matrix and a FFT matrix to be formed by using the retrieved parameters, thereby forming the second matrix information including the computed size and elements. The matrices formed in the matrix initializing section 441b may be identically established with those described in the first embodiment. Thus, detailed descriptions thereof will be omitted herein.

Although it is described as examples that the B-mode and the D-mode are selected above, the selection of the diagnostic mode is not limited thereto. The C-mode, the E-mode or the 3D-mode may be also selected. In this case, the linear operation matrix forming section 441b may also retrieve the linear operation information corresponding to the selected diagnostic mode from the mapping table to thereby form the linear operation matrix in the same manner as the B-mode or D-mode.

The texture data forming unit 441c may be operable to form first vertex data including the first matrix information provided from the matrix initializing section 441b and second vertex data including the second matrix information provided from the matrix initializing section 441b.

The data upload section 441d may be operable to allocate the storage areas of the video memory 441a according to the texture data corresponding to the selected diagnostic mode. In this case, the storage areas may include a storage area for storing the first texture data, a storage area for storing the second texture data and a storage area for storing the ultrasound data matrix. Also, the data upload section 441d may be further operable to upload the first and second texture data to the corresponding storage areas in the video memory 441a.

If the first and second texture data are uploaded to the video memory 441a, then the matrix forming section 441 e may be operable to form the ultrasound data matrix based on the first texture data. The formed ultrasound data matrix may be stored to the corresponding storage area in the video memory 441a. Also, the matrix forming section 441e may be further operable to perform matrix operations between the linear operations based on the second texture data to thereby form a final linear operation matrix. For example, if the B-mode is selected, then the matrix forming unit 441e may be operable to perform a matrix operation between a TGC matrix and a first FIR filter matrix, thereby obtaining a first matrix. The matrix forming unit 441e may be further operable to perform a matrix operation between the first matrix and a decimaltion matrix to thereby obtain a second matrix. The matrix forming unit 441e may be further operable to perform a matrix operation between the second matrix and a second FIR filter matrix to thereby obtain the final linear operation matrix. The first to final matrices may be stored in a temporal storage area in the video memory 441a.

Although the formation of the linear operation matrix is described with reference to the selection of the B-mode, when the D-mode, the C-mode, the E- mode or the 3D-mode is selected, the linear operation matrices may be also formed in the same manner as the selection of the B-mode.

Referring again to FIG. 11, the second processor 442 may be configured with one of CPU, FPGA, ASIC and DSP. The second processor 442 may be operable to perform a matrix operation between the ultrasound data matrix and the final linear operation matrix to thereby obtain ultrasound data with the linear operation performed. Also, the second processor 442 may be operable to perform a non-linear operation upon the obtained ultrasound data. Although it is described above that the second processor 442 may be operable to perform the non-linear operation after the linear operation in one embodiment, the non-linear operation may be performed prior to the linear operation in another embodiment.

The third processor 443 may be configured with GPU and is operable to perform data processing upon the ultrasound data with the linear and non-linear operations performed. The data processing may include at least one of scan conversion and rendering. The configuration and operation of the third processor 443 may be identical to those of the second processor 342 of the third embodiment. Thus, the detailed descriptions thereof will be omitted herein.

In one embodiment, although it is described that the first to third processors are separately configured, the configuration thereof is not limited thereto. The first to third processors may be embodied to a single processor such as GPU.

The display unit 450 may display an ultrasound image based on pixel data formed in the third processor 443. The control unit 460 may be operable to control the transmission/reception of the ultrasound signals, the formation of the ultrasound data, the linear operation and the non-linear operation. The control unit 460 may also control the display of the ultrasound image.

Fifth embodiment

FIG. 13 is a block diagram showing an illustrative embodiment of an ultrasound system 500. The ultrasound system 500 may include an input unit 510, an ultrasound data acquisition unit 520, a storage unit 530, a processing unit 540, a display unit 550 and a control unit 560. The configurations and operations of the input unit 510 and the ultrasound data acquisition unit 520 may be identical to those of the first embodiment. Thus, detailed descriptions thereof will be omitted herein. Also, the storage unit 530 may be identically configured with the storage unit 330 of the third embodiment. Accordingly, detailed descriptions will be omitted herein.

The processing unit 540 may be operable to form a linear operation matrix for performing linear operation upon the ultrasound data. The linear operation matrix may be formed by using the mapping table in response to an instruction for selecting a desired diagnostic mode, which is inputted through the input unit 510. The processing unit 540 may be further operable to perform linear operations corresponding to the selected diagnostic mode by using the linear operation matrix and non-linear operation upon the ultrasound data. In one embodiment, the non-linear operation may include envelope detection, compression, auto correlation, arctangent operation, a least mean square operation, a differential operation and the like. However, it is not limited thereto. The processing unit 540 may be operable to perform scan conversion or rendering upon the ultrasound data with the linear and non-linear operations performed.

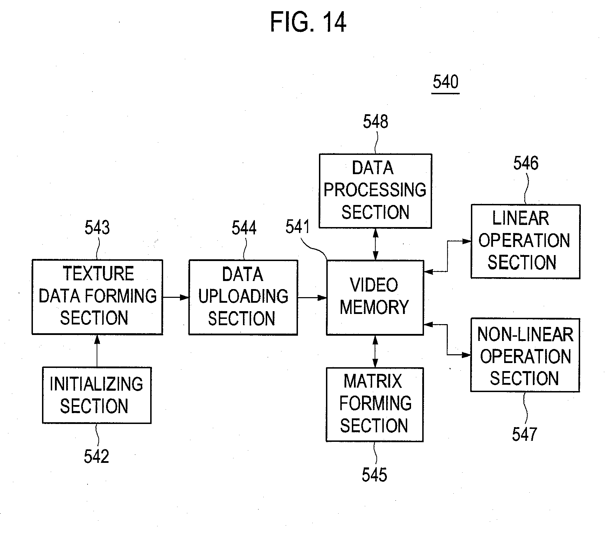

FIG. 14 is a block diagram showing an illustrative embodiment of the processing unit 540. The processing unit 540 may include a video memory 541 that may include a plurality of storage areas including a storage area for storing the pixel data corresponding to pixels consisting of a screen of the display unit 550. In one embodiment, the video memory 541 may be identically configured to the video memory 342a. Thus, detailed description thereof will be omitted herein.

The processing unit 540 may further include an initializing section 542. The initializing section 542 may be operable to form vertex information for initializing a position for displaying an ultrasound image on the screen of the display unit 550 by using a plurality of vertexes according to the selected diagnostic mode. In one embodiment, the vertex information may be identical to that of the third embodiment. Thus, detailed descriptions will be omitted herein.

The initializing section 542 may be operable to compute a size and elements of an ultrasound data matrix to be formed by using the ultrasound data, thereby forming first matrix information including the computed size and elements. Generally, the ultrasound data may be formed by scanning a plurality of scan lines and sampling electrical receive signals outputted in response to echo signals reflected from the scan lines. In one embodiment, since the ultrasound data may correspond to a plurality of sampling points on the scan lines, the ultrasound data matrix may be formed to have a size of the number of the sampling points the number of the scan lines.

The initializing section 542 may be operable to retrieve linear operation information corresponding to the diagnostic mode selected in response to the instruction inputted through the input unit 510 from the mapping table stored in the storage unit 530. The initializing section 542 may be further operable to compute a size and elements of a linear operation matrix to be formed by using the retrieved linear operation information, thereby forming second matrix information containing the computed size and elements.

The texture data forming section 543 may be operable to form first vertex data including the first matrix information provided from the initializing section 542 and second vertex data including the second matrix information provided from the initializing section 542. Further, the texture data forming section 543 may be operable to form third texture data including the vertex information provided from the initializing section 542.

The data upload section 544 may be operable to allocate the storage areas of the video memory 541 according to the first to third texture data corresponding to the selected diagnostic mode. In this case, the storage areas may include a storage area for storing the first texture data, a storage area for storing the second texture data, and a third storage area for storing the third texture data. The storage areas may further include a storage area for storing code data (e.g., shader code, field shader code and color palette data in case of the C-mode) necessary to perform functions of the GPU. Also, the data upload section 544 may be further operable to upload the first to third texture data to the corresponding storage areas in the video memory 541.

If the first to third texture data are uploaded to the video memory 541, then the matrix forming section 545 may be operable to form first ultrasound data matrix based on the first texture data. The formed first ultrasound data matrix may be stored to the corresponding storage area in the video memory 541. Also, the matrix forming section 545 may be further operable to perform matrix operations between the linear operations based on the second texture data to thereby form a final linear operation matrix. In one embodiment, the formation of the final linear operation may be identically performed to that of the final linear operation matrix in the matrix forming unit 441e of the fourth embodiment. Thus, detailed descriptions thereof will be omitted. The final matrices may be stored to a corresponding storage area in the video memory 541.

The linear operation section 546 may be operable to perform a matrix operation of the first ultrasound data matrix and the final linear operation matrix to thereby output second ultrasound data matrix. The outputted second ultrasound data matrix may be stored in a corresponding storage area in the video memory 541.

The non-linear operation section 547 may be operable to perform a matrix operation of the second ultrasound matrix and a non-linear operation matrix corresponding to the selected diagnostic mode to thereby output third ultrasound data matrix. The outputted third ultrasound data matrix may be stored to a corresponding storage area in the video memory 541.

Although it is described above that the linear operation is performed prior to the non-linear operation, the non-linear operation may be performed prior to the linear operation.

The data processing section 548 may be operable to perform data processing (e.g., scan conversion or rendering) upon the ultrasound data of the third ultrasound data matrix by using the shader codes uploaded to the video memory 541 according to the selected diagnostic mode. The data processing section 548 may be further operable to perform filtering, which applies the filter shader code to the data-processed ultrasound data, to thereby form the pixel data. The pixel data may be stored in a designated storage area in the video memory 342a.

Referring to FIG. 13, the display unit 550 may display an ultrasound image based on pixel data formed in the processing unit 540. The control unit 560 may be operable to control the transmission/reception of the ultrasound signals, the formation of the ultrasound data, the linear operation and the non-linear operation according to the selected diagnostic mode. The control unit 560 may also control the display of the ultrasound image.

As mentioned above, since the linear operations and the non-linear operations are separately performed to acquire an ultrasound image, an ultrasound data processing speed may be enhanced and the frame rate may be increased. Also, since the linear operations and the non-linear operations are performed in the GPU, the data processing speed may be also enhanced.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

**Claims**

1. An ultrasound system, comprising:

   a storage unit for storing a mapping table having linear operation information associated with a plurality of diagnostic modes;

   an input unit for allowing a user to input an instruction to select at least one of the diagnostic modes;

   an ultrasound data acquisition unit configured to transmit/receive ultrasound signals to/from a target object according to the selected diagnostic mode to thereby acquire ultrasound data; and

   a processing unit configured to form an ultrasound data matrix based on the ultrasound data and retrieve the linear operation information associated with the selected diagnostic mode from the mapping table to form linear operation matrices, the processing unit being further configured to perform a linear operation upon the ultrasound data matrix and the linear operation matrices and perform at least one non-linear operation upon the ultrasound data.

2. The system of Claim 1, wherein the processing unit is configured with one of a central processing unit, a field programmable gate array, an application specific integrated circuit, and a digital signal processing chip.

3. The system of Claim 2, wherein the linear operation information include time gain compensation (TGC) parameters, decimation parameters for adjusting amount of the ultrasound data, quadrature mixer parameters for forming inphase/quadrature-phase data, finite impulse response (FIR) filter parameters for FIR filtering, clutter filter parameters for clutter filtering, fast Fourier transform (FFT) parameters for FFT, scan conversion and interpolation parameters for scan conversion and interpolation, and rendering parameters for rendering.

4. The system of Claim 3, wherein the processing unit includes:

   an ultrasound data matrix forming section operable to form a first ultrasound data matrix based on the ultrasound data;

   a first linear operation matrix forming section operable to retrieve first linear operation information corresponding to the selected diagnostic mode from the mapping table to thereby form at least one first linear operation matrix;

   a first linear operation section operable to perform at least one first linear operation upon the first ultrasound data matrix and the first linear operation matrix to thereby output second ultrasound data matrix;

   a non-linear operation section operable to perform at least one non-linear operation upon the second ultrasound data matrix to output a third ultrasound data matrix;

   a second linear operation matrix forming section operable to retrieve second linear operation information corresponding to the selected diagnostic mode from the mapping table to thereby form at least one second linear operation matrix;

   a second linear operation section operable to perform at least one second linear operation upon the third ultrasound data matrix and the second linear operation matrix.

5. The ultrasound system of Claim 3, wherein the processing unit includes:

   an ultrasound data matrix forming section operable to form a first ultrasound data matrix based on the ultrasound data;

   a non-linear operation section operable to perform at least one non-linear operation upon the first ultrasound data matrix according to the selected diagnostic mode to thereby output a second ultrasound data matrix;

   a first linear operation matrix forming section operable to retrieve first linear operation information corresponding to the selected diagnostic mode from the mapping table to thereby form a first linear operation matrix;

   a first linear operation section operable to perform a matrix operation of the second ultrasound data matrix and the first linear operation matrix to thereby output third ultrasound data matrix;

   a second linear operation matrix forming section operable to retrieve second linear operation information corresponding to the selected diagnostic mode from the mapping table to thereby form a second linear operation matrix;

   a second linear operation section operable to perform a matrix operation of the third ultrasound data matrix and the second linear operation matrix.

**6.** The system of Claim 3, wherein the processing unit includes:

a first processor operable to perform at least one linear operation upon the ultrasound data by retrieving the linear operation information from the mapping table and at least one non-linear operation upon the ultrasound data with at least one linear operation performed; and
a second processor operable to perform scan conversion or rendering upon the ultrasound data with the linear and non-linear operations performed.

**7.** The system of Claim 6, wherein the first processor includes:

an ultrasound data matrix forming section operable to form a first ultrasound data matrix based on the ultrasound data;
a linear operation matrix forming section operable to retrieve linear operation information corresponding to the selected diagnostic mode from the mapping table to thereby form at least one linear operation matrix;
a linear operation section operable to perform at least one linear operation upon the first ultrasound data matrix and the linear operation matrix to thereby output second ultrasound data matrix; and
a non-linear operation section operable to perform at least one non-linear operation upon the second ultrasound data matrix according to the selected diagnostic mode.

**8.** The system of Claim 7, wherein the second processor includes:

a video memory containing a plurality of storage areas;
a vertex initializing section operable to initialize positions for displaying an ultrasound image by using a plurality vertexes according to the selected diagnostic mode to thereby form vertex information;
a texture data forming section operable to combine the ultrasound data with the linear and non-linear operations performed with the vertex information to thereby form texture data;
a data upload section operable to allocate the storage areas of the video memory according to a data format of the selected diagnostic mode and upload the texture data to a corresponding storage area; and
a data processing section operable to perform scan conversion or rendering upon the texture data and perform filtering upon the scan converted or rendered texture data to thereby form pixel data corresponding to the ultrasound image.

**9.** A method of processing ultrasound data in an ultrasound system, comprising:

a) storing a mapping table associating linear operation information with a plurality of diagnostic modes;
b) receiving an instruction to select at least one of the diagnostic mode;
c) transmitting/receiving ultrasound signals to/from a target object to thereby acquire ultrasound data according to the selected diagnostic mode; and
d) forming an ultrasound data matrix based on the ultrasound data, retrieving the linear operation information associated with the selected diagnostic mode from the mapping table to form linear operation matrices, and performing a linear operation upon the ultrasound data matrix and the linear operation matrices.

**10.** The method of Claim 9, further comprising performing at least one non-linear operation upon the ultrasound data.

**11.** The method of Claim 10, wherein the linear operation information include time gain compensation (TGC) parameters, decimation parameters for adjusting amount of the ultrasound data, quadrature mixer parameters for forming inphase/quadrature-phase data, finite impulse response (FIR) filter parameters for FIR filtering, clutter filter parameters for clutter filtering, fast Fourier transform (FFT) parameters for FFT, scan conversion and interpolation parameters for scan conversion and interpolation, and rendering parameters for rendering.

**12.** The method of Claim 11, wherein the step d) includes:

forming a first ultrasound data matrix based on the ultrasound data;
retrieving first linear operation information corresponding to the selected diagnostic mode from the mapping table to thereby form at least one first linear operation matrix;
performing at least one first linear operation upon the first ultrasound data matrix and the first linear operation matrix to thereby output second ultrasound data matrix;
performing at least one non-linear operation upon the second ultrasound data matrix to output a third ultrasound data matrix;

retrieving second linear operation information corresponding to the selected diagnostic mode from the mapping table to thereby form at least one second linear operation matrix; and

performing at least one second linear operation upon the third ultrasound data matrix and the second linear operation matrix.

**13.** The method of Claim 11, wherein the step d) includes:

forming a first ultrasound data matrix based on the ultrasound data;

performing at least one non-linear operation upon the first ultrasound data matrix according to the selected diagnostic mode to thereby output a second ultrasound data matrix;

retrieving first linear operation information corresponding to the selected diagnostic mode from the mapping table to thereby form a first linear operation matrix;

performing a matrix operation of the second ultrasound data matrix and the first linear operation matrix to thereby output third ultrasound data matrix; retrieving second linear operation information corresponding to the selected diagnostic mode from the mapping table to thereby form a second linear operation matrix; and

performing a matrix operation of the third ultrasound data matrix and the second linear operation matrix.

**14.** The method of Claim 11, wherein the step d) includes:

d1) performing at least one linear operation upon the ultrasound data by retrieving the linear operation information from the mapping table and at least one non-linear operation upon the ultrasound data with at least one linear operation performed; and

d2) performing scan conversion or rendering upon the ultrasound data with the linear and non-linear operations performed.

**15.** The method of Claim 14, wherein the step d1) includes:

forming a first ultrasound data matrix based on the ultrasound data;

retrieving linear operation information corresponding to the selected diagnostic mode from the mapping table to thereby form at least one linear operation matrix;

performing at least one linear operation upon the first ultrasound data matrix and the linear operation matrix to thereby output second ultrasound data matrix; and

performing at least one non-linear operation upon the second ultrasound data matrix according to the selected diagnostic mode, and

wherein the step d2) includes:

initializing positions for displaying an ultrasound image by using a plurality vertexes according to the selected diagnostic mode to thereby form vertex information;

combining the ultrasound data with the linear and non-linear operations performed with the vertex information to thereby form texture data;

allocating storage areas of a video memory according to a data format of the selected diagnostic mode and uploading the texture data to the corresponding storage area; and

performing scan conversion or rendering upon the texture data and performing filtering upon the scan converted or rendered texture data to thereby form pixel data corresponding to the ultrasound image.

EP 2 143 384 A1

FIG. 1

100

130          140

STORAGE UNIT          PROCESSING UNIT

120                                          160          150

ULTRASOUND ACQUISITION UNIT          CONTROL UNIT          DISPLAY UNIT

INPUT UNIT          110

FIG. 2

120

121          TX SIGNAL GENERATING SECTION

123                    124

122          ULTRASOUND PROBE          BEAM FORMER          ULTRASOUND DATA ACQUISITION SECTION

19

# FIG. 3

<u>140</u>

# FIG. 4

<u>200</u>

FIG. 5

_240_

241             243             244

| DATA MATRIX FORMING SECTION | → | LINEAR OPERATION SECTION | → | NON-LINEAR OPERATION SECTION |

LINEAR OPERATION MATRIX FORMING SECTION — 242

FIG. 6

_300_

341    340    342

FIRST PROCESSOR → SECOND PROCESSOR

320

ULTRASOUND DATA ACQUISITION UNIT ↔ CONTROL UNIT ↔ DISPLAY UNIT

360    350

INPUT UNIT      STORAGE UNIT

310          330

# FIG. 7

<u>341</u>

341a

DATA MATRIX
FORMING
SECTION

341c

LINEAR
OPERATION
SECTION

341d

NON-LINEAR
OPERATION
SECTION

LINEAR OPERATION
MATRIX FORMING
SECTION

341b

# FIG. 8

<u>342</u>

342c

TEXTURE DATA
ACQUISITION
SECTION

342d

DATA
UPLOADING
SECTION

342a

VIDEO
MEMORY

342e

DATA
PROCESSING
SECTION

VERTEX
INITIALIZING
SECTION

342b

# FIG. 9

# FIG. 10

VERTEX
INFORMATION
(A)

+

SCAN-CONVERTED
DATA
(B)

=

TEXTURE
DATA
(C)

# FIG. 11

EP 2 143 384 A1

# FIG. 12

<u>441</u>

441c

**TEXTURE DATA FORMING UNIT**

441d

**DATA UPLOADING UNIT**

441a

**VIDEO MEMORY**

441e

**MATRIX FORMING UNIT**

**MATRIX INITIALIZING UNIT**

441b

# FIG. 13

<u>500</u>

540

**PROCESSING UNIT**

520

**ULTRASOUND DATA ACQUISITION UNIT**

560

**CONTROL UNIT**

550

**DISPLAY UNIT**

**INPUT UNIT**

510

**STORAGE UNIT**

530

# FIG. 14

Europäisches
Patentamt

European
Patent Office

Office européen
des brevets

## EUROPEAN SEARCH REPORT

**Application Number**

EP 09 16 4024

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 383 139 B1 (HWANG JUIN-JET [US] ET AL) 7 May 2002 (2002-05-07) <br> * columns 8,6; figures 1,5-7 * | 1-15 | INV. <br> A61B8/08 <br> G01S15/89 <br> G06T11/00 <br> G06T15/00 <br> G06F9/302 |
| A | WO 2006/113445 A (VERASONICS INC [US]; DAIGLE RONALD ELVIN [US]) 26 October 2006 (2006-10-26) <br> * page 14, line 28 - page 17, line 18 * | 1,9 | |
| A | US 2007/161898 A1 (HAO XIAOHUI [US] ET AL) 12 July 2007 (2007-07-12) <br> * paragraphs [0058], [0059], [0065] * | 1,9 | |
| A | US 2008/161690 A1 (SATO TAKESHI [JP] ET AL) 3 July 2008 (2008-07-03) <br> * paragraphs [0050], [0061], [0070], [0071] * | 1,9 | |
| A | US 5 971 923 A (FINGER DAVID J [US]) 26 October 1999 (1999-10-26) <br> * column 18, lines 26-64 * | 1,9 | |
| A | US 5 881 178 A (TSYKALOV EUGENE N [US] ET AL) 9 March 1999 (1999-03-09) <br> * abstract; figure 1 * | 1,9 | TECHNICAL FIELDS SEARCHED (IPC) <br> A61B <br> G01S <br> G06T <br> G06F |
| A | US 2005/122333 A1 (SUMANAWEERA THILAKA S [US] ET AL) 9 June 2005 (2005-06-09) <br> * paragraph [0032] * | 1,9 | |
| A | US 2005/043619 A1 (SUMANAWEERA THILAKA [US] ET AL) 24 February 2005 (2005-02-24) <br> * paragraphs [0037], [0069], [0072] * | 1,9 | |

-/--

The present search report has been drawn up for all claims

| Place of search | Date of completion of the search | Examiner |
|---|---|---|
| Munich | 15 September 2009 | Anscombe, Marcel |

EPO FORM 1503 03.82 (P04C01)

Europäisches
Patentamt

European
Patent Office

Office européen
des brevets

# EUROPEAN SEARCH REPORT

Application Number

EP 09 16 4024

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JENSEN J L ET AL: "Multi-processor system for real-time deconvolution and flow estimation in medical ultrasound" ULTRASONICS SYMPOSIUM, 1996. PROCEEDINGS., 1996 IEEE SAN ANTONIO, TX, USA 3-6 NOV. 1996, IEEE, NEW YORK, NY, USA, vol. 2, 3 November 1996 (1996-11-03), pages 1197-1200, XP010217652 ISBN: 978-0-7803-3615-5 * page 1198, right-hand column, paragraph 1 * | 1,9 | |
| A | LATT J ET AL: "An implicitly parallel object-oriented matrix library and its application to medical physics" PARALLEL AND DISTRIBUTED PROCESSING SYMPOSIUM, 2003. PROCEEDINGS. INTERNATIONAL APRIL 22-26, 2003, PISCATAWAY, NJ, USA,IEEE, 22 April 2003 (2003-04-22), pages 254-261, XP010645514 ISBN: 978-0-7695-1926-5 Section 1.4.2 | 1,9 | |
| P,A | EP 2 058 672 A (MEDISON CO LTD [KR]) 13 May 2009 (2009-05-13) * the whole document * | 1,9 | TECHNICAL FIELDS SEARCHED (IPC) |

The present search report has been drawn up for all claims

| Place of search | Date of completion of the search | Examiner |
|---|---|---|
| Munich | 15 September 2009 | Anscombe, Marcel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 09 16 4024

This annex lists the patent family members relating to the patent documents cited in the above-mentioned European search report.
The members are as contained in the European Patent Office EDP file on
The European Patent Office is in no way liable for these particulars which are merely given for the purpose of information.

15-09-2009

| Patent document cited in search report | | Publication date | Patent family member(s) | | Publication date |
|---|---|---|---|---|---|
| US 6383139 | B1 | 07-05-2002 | US | 6416475 B1 | 09-07-2002 |
| WO 2006113445 | A | 26-10-2006 | CA | 2604649 A1 | 26-10-2006 |
| | | | CN | 101203183 A | 18-06-2008 |
| | | | EP | 1874192 A1 | 09-01-2008 |
| | | | JP | 2008536578 T | 11-09-2008 |
| | | | KR | 20080015082 A | 18-02-2008 |
| | | | US | 2009112095 A1 | 30-04-2009 |
| US 2007161898 | A1 | 12-07-2007 | WO | 2007081435 A1 | 19-07-2007 |
| US 2008161690 | A1 | 03-07-2008 | CN | 101209210 A | 02-07-2008 |
| | | | JP | 2008161289 A | 17-07-2008 |
| US 5971923 | A | 26-10-1999 | NONE | | |
| US 5881178 | A | 09-03-1999 | CA | 2190840 A1 | 30-11-1995 |
| | | | EP | 0764309 A1 | 26-03-1997 |
| | | | JP | 10501080 T | 27-01-1998 |
| | | | WO | 9532484 A1 | 30-11-1995 |
| US 2005122333 | A1 | 09-06-2005 | US | 2005140682 A1 | 30-06-2005 |
| US 2005043619 | A1 | 24-02-2005 | NONE | | |
| EP 2058672 | A | 13-05-2009 | JP | 2009119263 A | 04-06-2009 |
| | | | KR | 20090048243 A | 13-05-2009 |
| | | | US | 2009156934 A1 | 18-06-2009 |

For more details about this annex : see Official Journal of the European Patent Office, No. 12/82

**REFERENCES CITED IN THE DESCRIPTION**

**Patent documents cited in the description**

- KR 10200866718

- KR 102008106811